# EUROPEAN PATENT APPLICATION

(11) **EP 4 574 824 A1**
(43) Date of publication of application: **25.06.2025**
(21) Application number: 23854295.5
(22) Date of filing: 09.08.2023
(51) Int. Cl.: C07D 487/04, A61P 35/00, A61P 35/02

(54) **CRYSTAL FORM OF PYRAZOLOPYRIMIDINE ESTER COMPOUND AND PREPARATION METHOD THEREFOR**

(30) Priority: 17.08.2022 CN 202210984692
(71) Applicant: Shanghai Maius Pharmaceutical Co. Ltd., Shanghai 201210 (CN)
(72) Inventor: GUO, Yekun, Shanghai 201210 (CN); HUANG, Dujian, Shanghai 201210 (CN); HU, Xiang, Shanghai 201210 (CN); SHI, Mingfeng, Shanghai 201210 (CN)
(74) Representative: Plasseraud IP
(86) International application number: PCT/CN2023/111877
(87) International publication number: WO 2024/037392

(57) **Abstract**

The present invention provides a crystalline form Form I of a pyrazolopyrimidine ester compound of the structure of formula (I)

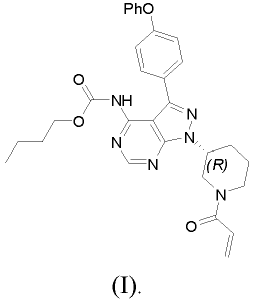

This crystalline form has been characterized using X-ray powder diffraction (XRPD), differential scanning calorimetry (DSC) and thermogravimetric analysis (TGA). The present invention also provides methods of preparing the crystalline form Form I of the pyrazolopyrimidine ester compound. This crystalline form allows long-term storage without special requirements in respect of temperature, light, humidity or oxygen presence and is significantly advantageous in terms of stability. Moreover, the methods involve simple steps and provide good reproducibility and excellent purity. Therefore, they have a promising prospect of extensive application.

## Description

### TECHNICAL FIELD

The present invention relates to the field of chemical medicines and, in particular, to a crystalline form of a pyrazolopyrimidine ester compound and methods of preparing the same.

### BACKGROUND

Bruton's tyrosine kinase (BTK) is a non-receptor cytoplasmic tyrosine protein kinase of the Tec family. BTK is involved in the transduction of TLR, BAFF-R, BCR and CXCR4/5 signals, as well as in the proliferation, differentiation, apoptosis and migration of regulatory B-cells. As BTK plays a crucial role in pathogenesis of malignant B-cell lymphoma, BTK inhibitors are used as important drugs for B-cell lymphoma treatment.

CN 202111131059.0 discloses a series of pyrazolopyrimidine ester compounds as BTK inhibitor prodrugs, which, once orally administered, will be rapidly absorbed into the blood and hydrolyzed into the active metabolite 1-[(3R)-3-[4-amino-3-(4-phenoxyphenyl)-1H-pyrazolo[3,4-d]pyrimidin-1-yl]-1-piperidinyl] -2-propen-1-onenamely the 4-amino active metabolite (4-AAM), an active metabolite commonly recognized in current clinical practice, to be able to exert the intended therapeutic effect. Among these compounds, the one chemically named as 1-[(3R)-3-[4-(butoxycarbonyl)-amino-3-(4-phenoxyphenyl)-1H-pyrazolo[3,4-d]pyrimidin-1-yl]-1-piperidinyl]-2-propen-1-one and of the chemical structure represented by formula (I) possesses the most desirable properties as a prodrug because it is hydrolyzed most rapidly in blood and has the highest 4-AAM AUC while itself showing the lowest AUC.

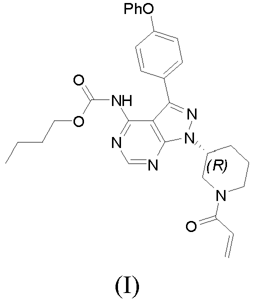

CN 202111131059.0 also provides a method for synthesis of this compound. A product obtained according to this method was found to be amorphous. The compound of formula (I) can be obtained by reacting commercially available 1-[(3R)-3-[4-amino-3-(4-phenoxyphenyl)-1H-pyrazolo[3,4-d]pyrimidin-1-yl]-1-piperidyl]-2-propen-1-one as one of the reactants with butyl chloroformate. Studies have shown that this amorphous compound exhibits poor stability and is susceptible to heat and moisture. Significant degradation can be observed after storage over 10 days at 40 °C, and almost complete degradation can be observed after storage over 10 days at 60 °C and 75% RH. Consequently, undesirable substances may emerge under regular storage conditions, creating great difficulties in the storage and processing of the product. Considering the potential clinical value of the compound of formula (I), it is particularly important to obtain its crystalline form with desirable purity and stable properties.

### SUMMARY

A stable and reproducible crystalline form is developed by the applicant and named as Form I.Also provided herein are methods of preparing Form I. The crystalline form Form I is significantly advantageous in terms of stability by allowing long-term storage without special requirements in respect of temperature, light, humidity or oxygen level.

It is a first object of the present invention to provide a crystalline form of a pyrazolopyrimidine ester compound. To this end, the present invention provides the technical solution as below:
A crystalline form Form I of a pyrazolopyrimidine ester compound of the structure of formula (I), which has three or more (preferably four or more, five or more, six or more, seven or more, or eight) characteristic peaks expressed in degrees 2θ at 11.26°, 14.03°, 14.80°, 17.07°, 19.78°, 21.21°, 22.39° and 23.85° in its X-ray powder diffraction (XRPD) pattern, wherein the error range of degrees 2θ is ± 0.20°, and the XRPD pattern is obtained using Cu-Kα radiation.

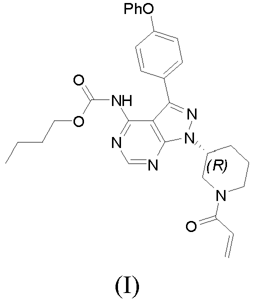

Additionally, the crystalline form Form I has characteristic peaks expressed in degrees 2θ at 6.99°, 8.62°, 10.72°, 11.05°, 11.26°, 11.86°, 12.07°, 13.08°, 14.03°, 14.80°, 16.28°, 17.07°, 19.33°, 19.78°, 20.19°, 20.69°, 21.21°, 22.39°, 22.86°, 23.08°, 23.85°, 24.40°, 24.73°, 25.95°, 26.23°, 26.95°, 29.21°, 30.17°, 32.71° and 32.99° in its XRPD pattern, wherein the error range of degrees 2θ is ± 0.20°.

Additionally, the degrees 2θ and relative intensities I (expressed as percentages to the most intense diffraction peak) of the crystalline form Form I in its XRPD pattern are as listed in the table below:

| 2θ ± 0.2 (°) | Relative Intensity I | 2θ ± 0.2 (°) | Relative Intensity I |
|---|---|---|---|
| 6.99 | 9.10% | 20.69 | 16.30% |
| 8.62 | 12.10% | 21.21 | 40.30% |
| 10.72 | 9.30% | 22.39 | 47.90% |
| 11.05 | 13.10% | 22.86 | 9.40% |
| 11.26 | 35.80% | 23.08 | 11.60% |
| 11.86 | 27.40% | 23.85 | 31.60% |
| 12.07 | 19.20% | 24.40 | 10.80% |
| 13.08 | 28.40% | 24.73 | 9.10% |
| 14.03 | 38.80% | 25.95 | 7.80% |
| 14.80 | 80.30% | 26.23 | 8.00% |
| 16.28 | 14.40% | 26.95 | 5.90% |
| 17.07 | 100.00% | 29.21 | 4.30% |
| 19.33 | 3.30% | 30.17 | 5.80% |
| 19.78 | 88.20% | 32.71 | 4.60% |
| 20.19 | 5.60% | 32.99 | 3.30% |

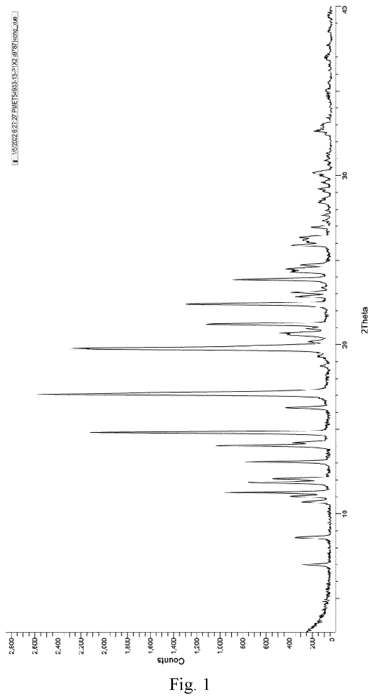

Additionally, the crystalline form Form I is characterized by the XRPD pattern as shown in Fig. 1.

Additionally, the crystalline form Form I is an irregularly shaped crystalline form.

Additionally, the crystalline form Form I has an endothermic peak at 119.75 °C in its differential scanning calorimetry (DSC) curve, wherein the error range of the endothermic peak temperature is ± 1.00 °C. In thermogravimetric analysis (TGA), the crystalline form Form I exhibits no significant weight loss before degradation, indicating that Form I is an anhydrous crystalline form.

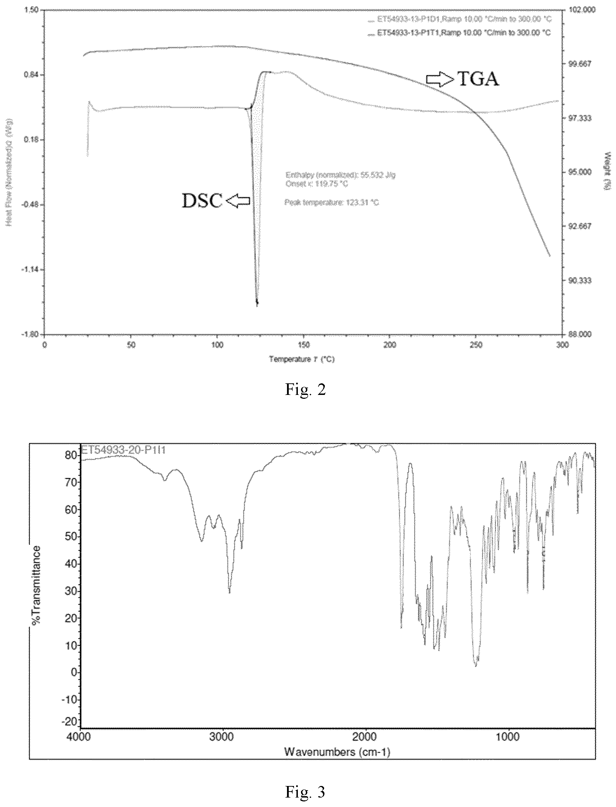

Additionally, the crystalline form Form I is characterized by the DSC and TGA curves as shown in Fig. 2.

Additionally, the crystalline form Form I has characteristic absorption bands at wavenumbers of 493, 519, 587, 609, 676, 692, 759, 774, 794, 808, 869, 895, 934, 963, 1000, 1027, 1073, 1102, 1134, 1158, 1229, 1342, 1382, 1448, 1490, 1523, 1559, 1589, 1645, 1681, 1753, 2870, 2954, 3067, 3149 and 3406 cm⁻¹ in its infrared (IR) spectrum, wherein the error range of the absorption band peaks is ± 2 cm⁻¹.

Additionally, the crystalline form Form I is characterized by IR spectrum as shown in Fig. 3.

Additionally, the crystalline form Form I is almost non-hygroscopic as determined by dynamic vapor sorption (DVS). The XRPD pattern of the crystalline form Form I remained the same before and after the DVS determination.

Additionally, the crystalline form Form I showed a lower degree of degradation than the amorphous form under several forced degradation conditions in a stability study. In particular, the crystalline form Form I samples were very stable over 10 days of storage at room temperature and in light. The crystalline form Form I can be stored under regular conditions without special protection as the study suggests, which is particularly important for pharmaceutical production.

It is a second object of the present invention to provide two methods of preparing the crystalline form Form I of the pyrazolopyrimidine ester compound as set forth above. Method I comprises the steps of:
adding the pyrazolopyrimidine ester compound of the structure of formula (I) in an amorphous form to a mixed solvent; and stirring the resulting suspension at room temperature, followed by filtering and drying, affording the pyrazolopyrimidine ester compound in the crystalline form Form I, wherein the mixed solvent is a solvent obtained by mixing a good solvent with a poor solvent;
wherein the good solvent is a solvent, in which the compound of formula (I) is soluble; and the poor solvent is a solvent, in which the compound of formula (I) is insoluble, sparingly soluble or slightly soluble.

Additionally, the good solvent in the mixed solvent is selected from methanol, ethanol, isopropanol, acetonitrile, acetone, butanone, ethyl acetate, isopropyl acetate and tetrahydrofuran, and the poor solvent is selected from methyl tert-butyl ether, water, n-heptane and cyclohexane.

Additionally, the mixed solvent is used at a volume-to-weight (v/w) ratio of 5 to 150 to the compound of formula (I), and in the mixed solvent, the poor solvent and the good solvent are used at a volume-to-volume (v/v) ratio of (1-14): 1, wherein v is measured in milliliters (mL), and w is measured in grams (g).

Method II comprises the steps of:
adding the pyrazolopyrimidine ester compound of the structure of formula (I) in an amorphous form to a pure solvent; and stirring the resulting suspension at room temperature, followed by filtering and drying, affording the pyrazolopyrimidine ester compound in the crystalline form Form I, wherein the pure solvent is selected from methanol, ethanol, isopropanol, isopropyl acetate, acetonitrile, methyl tert-butyl ether, n-heptane, isopropyl ether and cyclohexane.

Additionally, the pure solvent is used at a volume-to-weight (v/w) ratio of not exceeding 10, preferably of 10, to the compound of formula (I), wherein v is measured in milliliters (mL), and w is measured in grams (g).

It is a third object of the present invention to provide use of the crystalline form Form I of the pyrazolopyrimidine ester compound as set forth above for the preparation of medicaments for treating blood diseases such as lymphoma and lymphocytic leukemia.

The present invention offers the following benefits:
The pyrazolopyrimidine ester compound in the crystalline form Form I can be obtained according to the methods set forth in the present invention. This crystalline form allows long-term storage without special requirements in respect of temperature, light, humidity or oxygen level and is significantly superior over the amorphous form of pyrazolopyrimidine ester compound in terms of stability. In addition, the methods involve simple steps and provide good reproducibility and excellent purity. Therefore, they have a promising prospect of extensive application.

### BRIEF DESCRIPTION OF THE DRAWINGS

Fig. 1 shows an X-ray powder diffraction (XRPD) spectrum of a crystalline form Form I of a pyrazolopyrimidine ester compound according to the present invention.
Fig. 2 shows differential scanning calorimetry (DSC) and thermogravimetric analysis (TGA) curves of the crystalline form Form I of the pyrazolopyrimidine ester compound according to the present invention.
Fig. 3 shows an infrared (IR) spectrum of the crystalline form Form I of the pyrazolopyrimidine ester compound according to the present invention.

### DETAILED DESCRIPTION

The present invention will be described clearly and fully below with respect to specific embodiments thereof. It should be understood that the embodiments set forth herein are merely some, but not all, of the possible embodiments of this invention. Any and all other embodiments devisable by skilled artisans in light of the disclosed embodiments without paying any creative effort are considered to fall within the scope of the invention.

The amorphous compound of formula (I) used in the following examples can be prepared according to the method disclosed in CN202111131059.0: the reactants 1-[(3R)-3-[4-amino-3-(4-phenoxyphenyl)-1H-pyrazolo[3,4-d]pyrimidin-1-yl]-1-piperidyl]-2-propen-1-one, butyl chloroformate and dichloromethane were added under nitrogen protection; the mixture was cooled to 10 °C; pyridine was added dropwise and the reaction mixture was stirred for 3-4 hours at this temperature; ice water was added to quench the reaction when TLC shows that almost no reactants remain; aqueous phase was separated and extracted with dichloromethane; organic phases were combined and washed with water. The organic phase was concentrated and the residue was purified by column chromatography to afford the compound of formula (I).

In the context of the present invention, room temperature is defined as a temperature in the range of 20 to 30 °C, preferably 25 °C.

### Example 1: Preparation of Crystalline Form Form I According to Method I

50 mg of the amorphous compound of formula (I) was weighed and added to 0.5 mL of a mixed solvent. The mixed solvent was used at a ratio (v/w) of 10 to the compound of formula (I). The mixed solvent was obtained by mixing methyl tert-butyl ether (MTBE) as a poor solvent with ethanol as a good solvent at a volume-to-volume ratio of 9: 1. The resulting suspension was stirred at room temperature for 3 days and then filtered, affording a solid which was then dried and subjected to XRPD, TGA and DSC analysis. The results showed that the pyrazolopyrimidine ester compound was obtained in the crystalline form Form I.

### Example 2: Preparation of Crystalline Form Form I According to Method I

50 mg of the amorphous compound of formula (I) was weighed and added to 0.5 mL of a mixed solvent. The mixed solvent was used at a ratio (v/w) of 10 to the compound of formula (I). The mixed solvent was obtained by mixing MTBE as a poor solvent with tetrahydrofuran (THF) as a good solvent at a volume-to-volume ratio of 9: 1. The resulting suspension was stirred at room temperature for 3 days and then filtered, affording a solid which was then dried and subjected to XRPD analysis. The results showed that the pyrazolopyrimidine ester compound was obtained in the crystalline form Form I.

### Example 3: Preparation of Crystalline Form Form I According to Method I

50 mg of the amorphous compound of formula (I) was weighed and added to 0.5 mL of a mixed solvent. The mixed solvent was used at a ratio (v/w) of 10 to the compound of formula (I). The mixed solvent was obtained by mixing MTBE as a poor solvent with isopropyl acetate as a good solvent at a volume-to-volume ratio of 9: 1. The resulting suspension was stirred at room temperature for 3 days and then filtered, affording a solid which was then dried and subjected to XRPD analysis. The results showed that the pyrazolopyrimidine ester compound was obtained in the crystalline form Form I.

### Example 4: Preparation of Crystalline Form Form I According to Method I

50 mg of the amorphous compound of formula (I) was weighed and added to 0.5 mL of a mixed solvent. The mixed solvent was used at a ratio (v/w) of 10 to the compound of formula (I). The mixed solvent was obtained by mixing MTBE as a poor solvent with ethyl acetate as a good solvent at a volume-to-volume ratio of 9: 1. The resulting suspension was stirred at room temperature for 3 days and then filtered, affording a solid which was then dried and subjected to XRPD analysis. The results showed that the pyrazolopyrimidine ester compound was obtained in the crystalline form Form I.

### Example 5: Preparation of Crystalline Form Form I According to Method I

50 mg of the amorphous compound of formula (I) was weighed and added to 0.5 mL of a mixed solvent. The mixed solvent was used at a ratio (v/w) of 10 to the compound of formula (I). The mixed solvent was obtained by mixing MTBE as a poor solvent with acetonitrile as a good solvent at a volume-to-volume ratio of 9: 1. The resulting suspension was stirred at room temperature for 3 days and then filtered, affording a solid which was then dried and subjected to XRPD analysis. The results showed that the pyrazolopyrimidine ester compound was obtained in the crystalline form Form I.

### Example 6: Preparation of Crystalline Form Form I According to Method I

50 mg of the amorphous compound of formula (I) was weighed and added to 0.5 mL of a mixed solvent. The mixed solvent was used at a ratio (v/w) of 10 to the compound of formula (I). The mixed solvent was obtained by mixing MTBE as a poor solvent with ethanol as a good solvent at a volume-to-volume ratio of 7: 1. The resulting suspension was stirred at room temperature for 3 days and then filtered, affording a solid which was then dried and subjected to XRPD analysis. The results showed that the pyrazolopyrimidine ester compound was obtained in the crystalline form Form I.

### Example 7: Preparation of Crystalline Form Form I According to Method I

50 mg of the amorphous compound of formula (I) was weighed and added to 0.5 mL of a mixed solvent. The mixed solvent was used at a ratio (v/w) of 10 to the compound of formula (I). The mixed solvent was obtained by mixing n-heptane as a poor solvent with THF as a good solvent at a volume-to-volume ratio of 2: 1. The resulting suspension was stirred at room temperature for 3 days and then filtered, affording a solid which was then dried and subjected to XRPD analysis. The results showed that the pyrazolopyrimidine ester compound was obtained in the crystalline form Form I.

### Example 8: Preparation of Crystalline Form Form I According to Method I

50 mg of the amorphous compound of formula (I) was weighed and added to 0.5 mL of a mixed solvent. The mixed solvent was used at a ratio (v/w) of 10 to the compound of formula (I). The mixed solvent was obtained by mixing n-heptane as a poor solvent with isopropyl acetate as a good solvent at a volume-to-volume ratio of 2: 1. The resulting suspension was stirred at room temperature for 3 days and then filtered, affording a solid which was then dried and subjected to XRPD analysis. The results showed that the pyrazolopyrimidine ester compound was obtained in the crystalline form Form I.

### Example 9: Preparation of Crystalline Form Form I According to Method I

50 mg of the amorphous compound of formula (I) was weighed and added to 0.5 mL of a mixed solvent. The mixed solvent was used at a ratio (v/w) of 10 to the compound of formula (I). The mixed solvent was obtained by mixing n-heptane as a poor solvent with ethyl acetate as a good solvent at a volume-to-volume ratio of 2: 1. The resulting suspension was stirred at room temperature for 3 days and then filtered, affording a solid which was then dried and subjected to XRPD analysis. The results showed that the pyrazolopyrimidine ester compound was obtained in the crystalline form Form I.

### Example 10: Preparation of Crystalline Form Form I According to Method I

50 mg of the amorphous compound of formula (I) was weighed and added to 0.5 mL of a mixed solvent. The mixed solvent was used at a ratio (v/w) of 10 to the compound of formula (I). The mixed solvent was obtained by mixing n-heptane as a poor solvent with acetone as a good solvent at a volume-to-volume ratio of 2: 1. The resulting suspension was stirred at room temperature for 3 days and then filtered, affording a solid which was then dried and subjected to XRPD analysis. The results showed that the pyrazolopyrimidine ester compound was obtained in the crystalline form Form I.

### Example 11: Preparation of Crystalline Form Form I According to Method I

50 mg of the amorphous compound of formula (I) was weighed and added to 0.5 mL of a mixed solvent. The mixed solvent was used at a ratio (v/w) of 10 to the compound of formula (I). The mixed solvent was obtained by mixing water as a poor solvent with methanol as a good solvent at a volume-to-volume ratio of 2: 1. The resulting suspension was stirred at room temperature for 3 days and then filtered, affording a solid which was then dried and subjected to XRPD analysis. The results showed that the pyrazolopyrimidine ester compound was obtained in the crystalline form Form I.

### Example 12: Preparation of Crystalline Form Form I According to Method I

50 mg of the amorphous compound of formula (I) was weighed and added to 0.5 mL of a mixed solvent. The mixed solvent was used at a ratio (v/w) of 10 to the compound of formula (I). The mixed solvent was obtained by mixing water as a poor solvent with butanone as a good solvent at a volume-to-volume ratio of 2: 1. The resulting suspension was stirred at room temperature for 3 days and then filtered, affording a solid which was then dried and subjected to XRPD analysis. The results showed that the pyrazolopyrimidine ester compound was obtained in the crystalline form Form I.

### Example 13: Preparation of Crystalline Form Form I According to Method I

50 mg of the amorphous compound of formula (I) was weighed and added to 0.5 mL of a mixed solvent. The mixed solvent was used at a ratio (v/w) of 10 to the compound of formula (I). The mixed solvent was obtained by mixing water as a poor solvent with acetonitrile as a good solvent at a volume-to-volume ratio of 2: 1. The resulting suspension was stirred at room temperature for 3 days and then filtered, affording a solid which was then dried and subjected to XRPD analysis. The results showed that the pyrazolopyrimidine ester compound was obtained in the crystalline form Form I.

### Example 14: Preparation of Crystalline Form Form I According to Method I

50 mg of the amorphous compound of formula (I) was weighed and added to 0.5 mL of a mixed solvent. The mixed solvent was used at a ratio (v/w) of 10 to the compound of formula (I). The mixed solvent was obtained by mixing water as a poor solvent with isopropanol as a good solvent at a volume-to-volume ratio of 2: 1. The resulting suspension was stirred at room temperature for 3 days and then filtered, affording a solid which was then dried and subjected to XRPD analysis. The results showed that the pyrazolopyrimidine ester compound was obtained in the crystalline form Form I.

### Example 15: Preparation of Crystalline Form Form I According to Method I

50 mg of the amorphous compound of formula (I) was weighed and added to 0.75 mL of a mixed solvent. The mixed solvent was used at a ratio (v/w) of 15 to the compound of formula (I). The mixed solvent was obtained by mixing cyclohexane as a poor solvent with acetone as a good solvent at a volume-to-volume ratio of 9: 1. The resulting suspension was stirred at room temperature for 3 days and then filtered, affording a solid which was then dried and subjected to XRPD analysis. The results showed that the pyrazolopyrimidine ester compound was obtained in the crystalline form Form I.

### Example 16: Preparation of Crystalline Form Form I According to Method I

50 mg of the amorphous compound of formula (I) was weighed and added to 0.75 mL of a mixed solvent. The mixed solvent was used at a ratio (v/w) of 15 to the compound of formula (I). The mixed solvent was obtained by mixing cyclohexane as a poor solvent with butanone as a good solvent at a volume-to-volume ratio of 9: 1. The resulting suspension was stirred at room temperature for 3 days and then filtered, affording a solid which was then dried and subjected to XRPD analysis. The results showed that the pyrazolopyrimidine ester compound was obtained in the crystalline form Form I.

### Example 17: Preparation of Crystalline Form Form I According to Method I

50 mg of the amorphous compound of formula (I) was weighed and added to 0.25 mL of a mixed solvent. The mixed solvent was used at a ratio (v/w) of 5 to the compound of formula (I). The mixed solvent was obtained by mixing cyclohexane as a poor solvent with isopropanol as a good solvent at a volume-to-volume ratio of 9: 1. The resulting suspension was stirred at room temperature for 3 days and then filtered, affording a solid which was then dried and subjected to XRPD analysis. The results showed that the pyrazolopyrimidine ester compound was obtained in the crystalline form Form I.

### Example 18: Preparation of Crystalline Form Form I According to Method I

50 mg of the amorphous compound of formula (I) was weighed and added to 3.5 mL of a mixed solvent. The mixed solvent was used at a ratio (v/w) of 70 to the compound of formula (I). The mixed solvent was obtained by mixing n-heptane as a poor solvent with acetone as a good solvent at a volume-to-volume ratio of 6: 1. The resulting suspension was stirred at room temperature for 3 days and then filtered, affording a solid which was then dried and subjected to XRPD analysis. The results showed that the pyrazolopyrimidine ester compound was obtained in the crystalline form Form I.

### Example 19: Preparation of Crystalline Form Form I According to Method I

50 mg of the amorphous compound of formula (I) was weighed and added to 7.5 mL of a mixed solvent. The mixed solvent was used at a ratio (v/w) of 150 to the compound of formula (I). The mixed solvent was obtained by mixing cyclohexane as a poor solvent with acetone as a good solvent at a volume-to-volume ratio of 14: 1. The resulting suspension was stirred at room temperature for 3 days and then filtered, affording a solid which was then dried and subjected to XRPD analysis. The results showed that the pyrazolopyrimidine ester compound was obtained in the crystalline form Form I.

### Example 20: Preparation of Crystalline Form Form I According to Method I

50 mg of the amorphous compound of formula (I) was weighed and added to 2.0 mL of a mixed solvent. The mixed solvent was used at a ratio (v/w) of 40 to the compound of formula (I). The mixed solvent was obtained by mixing water as a poor solvent with acetonitrile as a good solvent at a volume-to-volume ratio of 1: 1. The resulting suspension was stirred at room temperature for 3 days and then filtered, affording a solid which was then dried and subjected to XRPD analysis. The results showed that the pyrazolopyrimidine ester compound was obtained in the crystalline form Form I.

### Example 21: Preparation of Crystalline Form Form I According to Method I

50 mg of the amorphous compound of formula (I) was weighed and added to 4.0 mL of a mixed solvent. The mixed solvent was used at a ratio (v/w) of 80 to the compound of formula (I). The mixed solvent was obtained by mixing n-heptane as a poor solvent with ethyl acetate as a good solvent at a volume-to-volume ratio of 7: 1. The resulting suspension was stirred at room temperature for 3 days and then filtered, affording a solid which was then dried and subjected to XRPD analysis. The results showed that the pyrazolopyrimidine ester compound was obtained in the crystalline form Form I.

### Example 22: Preparation of Crystalline Form Form I According to Method I

50 mg of the amorphous compound of formula (I) was weighed and added to 7.5 mL of a mixed solvent. The mixed solvent was used at a ratio (v/w) of 150 to the compound of formula (I). The mixed solvent was obtained by mixing cyclohexane as a poor solvent with ethyl acetate as a good solvent at a volume-to-volume ratio of 14: 1. The resulting suspension was stirred at room temperature for 3 days and then filtered, affording a solid which was then dried and subjected to XRPD analysis. The results showed that the pyrazolopyrimidine ester compound was obtained in the crystalline form Form I.

### Example 23: Preparation of Crystalline Form Form I According to Method I

50 mg of the amorphous compound of formula (I) was weighed and added to 3.5 mL of a mixed solvent. The mixed solvent was used at a ratio (v/w) of 70 to the compound of formula (I). The mixed solvent was obtained by mixing n-heptane as a poor solvent with THF as a good solvent at a volume-to-volume ratio of 6: 1. The resulting suspension was stirred at room temperature for 3 days and then filtered, affording a solid which was then dried and subjected to XRPD analysis. The results showed that the pyrazolopyrimidine ester compound was obtained in the crystalline form Form I.

### Example 24: Preparation of Crystalline Form Form I According to Method I

50 mg of the amorphous compound of formula (I) was weighed and added to 7.5 mL of a mixed solvent. The mixed solvent was used at a ratio (v/w) of 150 to the compound of formula (I). The mixed solvent was obtained by mixing cyclohexane as a poor solvent with THF as a good solvent at a volume-to-volume ratio of 14: 1. The resulting suspension was stirred at room temperature for 3 days and then filtered, affording a solid which was then dried and subjected to XRPD analysis. The results showed that the pyrazolopyrimidine ester compound was obtained in the crystalline form Form I.

### Example 25: Preparation of Crystalline Form Form I According to Method II

50 mg of the amorphous compound of formula (I) was weighed and added to 0.5 mL of a pure solvent. The pure solvent was methanol and used at a ratio (v/w) of 10 to the compound of formula (I). The resulting suspension was stirred at room temperature for 3 days and then filtered, affording a solid which was then dried and subjected to XRPD analysis. The results showed that the pyrazolopyrimidine ester compound was obtained in the crystalline form Form I.

### Example 26: Preparation of Crystalline Form Form I According to Method II

50 mg of the amorphous compound of formula (I) was weighed and added to 0.5 mL of a pure solvent. The pure solvent was ethanol and used at a ratio (v/w) of 10 to the compound of formula (I). The resulting suspension was stirred at room temperature for 3 days and then filtered, affording a solid which was then dried and subjected to XRPD analysis. The results showed that the pyrazolopyrimidine ester compound was obtained in the crystalline form Form I.

### Example 27: Preparation of Crystalline Form Form I According to Method II

50 mg of the amorphous compound of formula (I) was weighed and added to 0.5 mL of a pure solvent. The pure solvent was isopropanol and used at a ratio (v/w) of 10 to the compound of formula (I). The resulting suspension was stirred at room temperature for 3 days and then filtered, affording a solid which was then dried and subjected to XRPD analysis. The results showed that the pyrazolopyrimidine ester compound was obtained in the crystalline form Form I.

### Example 28: Preparation of Crystalline Form Form I According to Method II

50 mg of the amorphous compound of formula (I) was weighed and added to 0.5 mL of a pure solvent. The pure solvent was isopropyl acetate and used at a ratio (v/w) of 10 to the compound of formula (I). The resulting suspension was stirred at room temperature for 3 days and then filtered, affording a solid which was then dried and subjected to XRPD analysis. The results showed that the pyrazolopyrimidine ester compound was obtained in the crystalline form Form I.

### Example 29: Preparation of Crystalline Form Form I According to Method II

50 mg of the amorphous compound of formula (I) was weighed and added to 0.5 mL of a pure solvent. The pure solvent was acetonitrile and used at a ratio (v/w) of 10 to the compound of formula (I). The resulting suspension was stirred at room temperature for 3 days and then filtered, affording a solid which was then dried and subjected to XRPD analysis. The results showed that the pyrazolopyrimidine ester compound was obtained in the crystalline form Form I.

### Example 30: Preparation of Crystalline Form Form I According to Method II

50 mg of the amorphous compound of formula (I) was weighed and added to 0.5 mL of a pure solvent. The pure solvent was MTBE and used at a ratio (v/w) of 10 to the compound of formula (I). The resulting suspension was stirred at room temperature for 3 days and then filtered, affording a solid which was then dried and subjected to XRPD analysis. The results showed that the pyrazolopyrimidine ester compound was obtained in the crystalline form Form I.

### Example 31: Preparation of Crystalline Form Form I According to Method II

50 mg of the amorphous compound of formula (I) was weighed and added to 0.5 mL of a pure solvent. The pure solvent was n-heptane and used at a ratio (v/w) of 10 to the compound of formula (I). The resulting suspension was stirred at room temperature for 3 days and then filtered, affording a solid which was then dried and subjected to XRPD analysis. The results showed that the pyrazolopyrimidine ester compound was obtained in the crystalline form Form I.

### Example 32: Preparation of Crystalline Form Form I According to Method II

50 mg of the amorphous compound of formula (I) was weighed and added to 0.5 mL of a pure solvent. The pure solvent was isopropyl ether and used at a ratio (v/w) of 10 to the compound of formula (I). The resulting suspension was stirred at room temperature for 3 days and then filtered, affording a solid which was then dried and subjected to XRPD analysis. The results showed that the pyrazolopyrimidine ester compound was obtained in the crystalline form Form I.

### Example 33: Preparation of Crystalline Form Form I According to Method II

50 mg of the amorphous compound of formula (I) was weighed and added to 0.5 mL of a pure solvent. The pure solvent was cyclohexane and used at a ratio (v/w) of 10 to the compound of formula (I). The resulting suspension was stirred at room temperature for 3 days and then filtered, affording a solid which was then dried and subjected to XRPD analysis. The results showed that the pyrazolopyrimidine ester compound was obtained in the crystalline form Form I.

### Comparative Example 1

50 mg of the amorphous compound of formula (I) was weighed and added to 0.5 mL of a pure solvent. The pure solvent was water. The resulting suspension was stirred at room temperature for 3 days and then filtered, affording a solid which was then dried and subjected to XRPD analysis. The results showed that the resulting solid was amorphous.

### Comparative Example 2

50 mg of the amorphous compound of formula (I) was weighed and added to 0.5 mL of a pure solvent. The pure solvent was ethyl acetate. The resulting suspension still appeared as a clear solution with no solid precipitate present, after being stirred at room temperature for 3 days.

### Comparative Example 3

50 mg of the amorphous compound of formula (I) was weighed and added to 0.5 mL of a pure solvent. The pure solvent was acetone. The resulting suspension still appeared as a clear solution with no solid precipitate present, after being stirred at room temperature for 3 days.

### Example 34: Stability Study

Samples of the amorphous and crystalline (Form I) compound of formula (I) were stored under different conditions: at 40 °C for 10 days; at 150 °C for 10 days; at 25 °C and 92.5% RH (relative humidity) for 10 days; at 60 °C and 75% RH for 10 days; in light for 10 days; and at room temperature for 10 days. Changes in purity of them were then analyzed, and the results are shown in the table below.

Noticeable degradation of the amorphous sample that had been stored at 40 °C for 10 days and almost complete degradation of the amorphous sample that had been stored at 60°C and 75% RH for 10 days were observed. Form I samples showed lower degrees of degradation than the amorphous compound, in particular when stored at room temperature and in light for 10 days, remaining highly stable. The crystalline form Form I has demonstrated its value in terms of stability - it allows long-term storage without special requirements in respect of temperature, light, humidity or oxygen level.

| | Initial (Day 0) | 40°C (Day 10) | 105°C (Day 10) | 25°C + 92.5% RH (Day 10) | 60°C + 75% RH (Day 10) | In Light (Day 10) | Room Temperature (Day 10) |
|---|---|---|---|---|---|---|---|
| Amorphous | 99.07% | 96.10% | 0 | 98.00% | 0.27% | 98.63% | 98.01% |
| Form I | 98.68% | 98.60% | 2.19% | 98.63% | 90.62% | 98.62% | 98.64% |

## Claims

1. A crystalline form Form I of a pyrazolopyrimidine ester compound of the structure of formula (I), **characterized by** 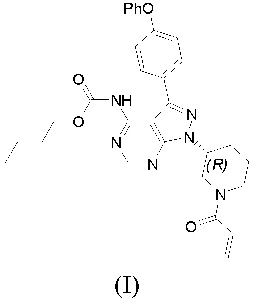 having characteristic peaks expressed in degrees 2θ at 11.26°, 14.03°, 14.80°, 17.07°, 19.78°, 21.21°, 22.39° and 23.85° in its X-ray powder diffraction (XRPD) pattern, wherein the error range of degrees 2θ is ± 0.20°, and the XRPD pattern is obtained using Cu-Kα radiation.

2. The crystalline form Form I of the pyrazolopyrimidine ester compound according to claim 1, **characterized by** having characteristic peaks expressed in degrees 2θ values at 6.99°, 8.62°, 10.72°, 11.05°, 11.26°, 11.86°, 12.07°, 13.08°, 14.03°, 14.80°, 16.28°, 17.07°, 19.33°, 19.78°, 20.19°, 20.69°, 21.21°, 22.39°, 22.86°, 23.08°, 23.85°, 24.40°, 24.73°, 25.95°, 26.23°, 26.95°, 29.21°, 30.17°, 32.71° and 32.99° in the XRPD pattern, wherein the error range of degrees 2θ is ± 0.20°.

3. The crystalline form Form I of the pyrazolopyrimidine ester compound according to claim 2, **characterized by** the XRPD pattern as shown in Fig. 1.

4. The crystalline form Form I of the pyrazolopyrimidine ester compound according to claim 1, **characterized by** being an irregularly shaped and anhydrous crystalline formand having an endothermic peak at 119.75 °C in its differential scanning calorimetry (DSC) curve, wherein the error range of the endothermic peak temperature is ± 1.00 °C.

5. The crystalline form Form I of the pyrazolopyrimidine ester compound according to claim 4, **characterized by** the DSC and thermogravimetric analysis (TGA) curves as shown in Fig. 2.

6. The crystalline form Form I of the pyrazolopyrimidine ester compound according to claim 1, **characterized by** having characteristic absorption bands at wavenumbers of 493, 519, 587, 609, 676, 692, 759, 774, 794, 808, 869, 895, 934, 963, 1000, 1027, 1073, 1102, 1134, 1158, 1229, 1342, 1382, 1448, 1490, 1523, 1559, 1589, 1645, 1681, 1753, 2870, 2954, 3067, 3149 and 3406 cm⁻¹ in its infrared (IR) spectrum, wherein the error range of the absorption band peaks is ± 2 cm⁻¹.

7. The crystalline form Form I of the pyrazolopyrimidine ester compound according to claim 6, **characterized by** the IR spectrum as shown in Fig. 3.

8. A method of preparing the crystalline form Form I of the pyrazolopyrimidine ester compound according to any of claims 1 to 7, **characterized in** comprising the steps of:
adding the pyrazolopyrimidine ester compound of the structure of formula (I) in an amorphous form to a mixed solvent; and stirring the resulting suspension, followed by filtering and drying, affording the pyrazolopyrimidine ester compound in the crystalline form Form I, wherein the mixed solvent is a solvent obtained by mixing a good solvent with a poor solvent;
wherein the good solvent in the mixed solvent is selected from methanol, ethanol, isopropanol, acetonitrile, acetone, butanone, ethyl acetate, isopropyl acetate and tetrahydrofuran; the poor solvent is selected from methyl tert-butyl ether, water, n-heptane and cyclohexane; and the mixed solvent is used at a volume-to-weight ratio of 5 to 150 to the compound of formula (I), and in the mixed solvent, the poor solvent and the good solvent are used at a volume-to-volume ratio of (1-14) :1.

9. A method of preparing the crystalline form Form I of the pyrazolopyrimidine ester compound according to any of claims 1 to 7, **characterized in** comprising the steps of:
adding the pyrazolopyrimidine ester compound of the structure of formula (I) in an amorphous form to a pure solvent; and stirring the resulting suspension, followed by filtering and drying, affording the pyrazolopyrimidine ester compound in the crystalline form Form I, wherein the pure solvent is selected from methanol, ethanol, isopropanol, isopropyl acetate, acetonitrile, methyl tert-butyl ether, n-heptane, isopropyl ether and cyclohexane; and
the pure solvent is used at a volume-to-weight ratio of not exceeding 10 to the compound of formula (I).

10. Use of the crystalline form Form I of the pyrazolopyrimidine ester compound according to any of claims 1 to 7, **characterized in that** it is used to prepare a medicament for treating lymphoma and lymphocytic leukemia.
